(19) Europäisches Patentamt
European Patent Office
Office européen des brevets

(11) EP 0 395 323 B1

(12) **EUROPEAN PATENT SPECIFICATION**

(45) Date of publication and mention of the grant of the patent:
**11.06.1997 Bulletin 1997/24**

(51) Int Cl.[6]: **C07D 281/10**
// A61K31/55

(21) Application number: **90304299.2**

(22) Date of filing: **20.04.1990**

(54) **Process for preparing 1,5-benzothiazepine derivatives**

Verfahren zur Herstellung von 1,5-Benzothiazepin-Derivaten

Procédé pour la préparation de dérivés de 1,5-benzothiazépine

(84) Designated Contracting States:
**AT BE CH DE DK ES GB GR IT LI LU NL SE**

(30) Priority: **28.04.1989 JP 109795/89**

(43) Date of publication of application:
**31.10.1990 Bulletin 1990/44**

(73) Proprietor: **TANABE SEIYAKU CO., LTD.**
**Chuo-ku Osaka (JP)**

(72) Inventors:
- **Hayashi, Hironori**
  **Toyonaka-shi, Osaka-fu (JP)**
- **Nishimoto, Shigeru**
  **Minoo-shi, Osaka-ku (JP)**
- **Okuno, Tamotsu**
  **Kawanishi-shi, Hyogo-ken (JP)**
- **Kitano, Masashi**
  **Kitakatsuragi-gun, Nara-ken (JP)**
- **Maeda, Sadao**
  **Ibaraki-shi, Osaka-fu (JP)**

(74) Representative:
**Baverstock, Michael George Douglas et al**
**BOULT WADE TENNANT**
**27 Furnival Street**
**London, EC4A 1PQ (GB)**

(56) References cited:
**EP-A- 0 098 422**
**EP-A- 0 343 474**
**EP-A- 0 378 455**
**GB-A- 2 139 620**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 395 323 B1

Printed by Jouve, 75001 PARIS (FR)

## Description

This invention relates to a novel process for preparing 1,5-benzothiazepine derivatives represented by the formula:

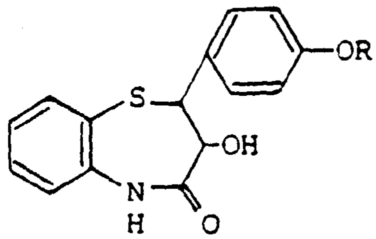

(I)

wherein R represents a $C_1$-$C_4$ alkyl group.

The above 1,5-benzothiazepine derivatives (I) are useful compounds as intermediates for the synthesis of medical compounds, for example, (+)-cis-2-(4-methoxyphenyl)-3-acetoxy-5-(β-dimethylaminoethyl)-2,3-dihydro-1,5-benzothiazepin-4(5H)-one (generic name: Diltiazem) having excellent coronary vasodilating activity.

Heretofore, as a process for preparing 1,5-benzothiazepine derivatives (I), for example, the method in which 2-hydroxy-3-(2-aminophenylthio)-3-(4-methoxyphenyl)propionic acid is subjected to intramolecular ring closing reaction in xylene under reflux (Japanese Patent Publication No. 18038/1978) has been known. However, this method is disadvantageous in that a long time (12 hours in Examples of the above publication is required for completing the reaction.

EP-A-343 474 and EP-A-378 455 are comprised in the state of the art in accordance with Article 54(3) EPC.

Furthermore, EP-A-343 474 (which was published after the Japanese Priority date of this present application) relates to a process for preparing an acid of the general formula

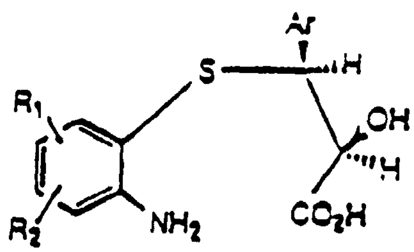

A

wherein $R_1$ and $R_2$ are each, independently, hydrogen, alkyl of 1 to 4 carbon atoms, alkoxy of l to 4 carbon atoms, halogen, trifluoromethyl or nitro or $R_1$ and $R_2$ taken together with the benzene ring to which they are attached, are naphthalene and Ar is phenyl which unsubstituted or substituted by one to three substituents selected from the group consisting of alkyl of 1 to 4 carbon atoms, alkoxy of 1 to 4 carbon atoms and halogen, which comprises hydrolyzing a compound of the general formula

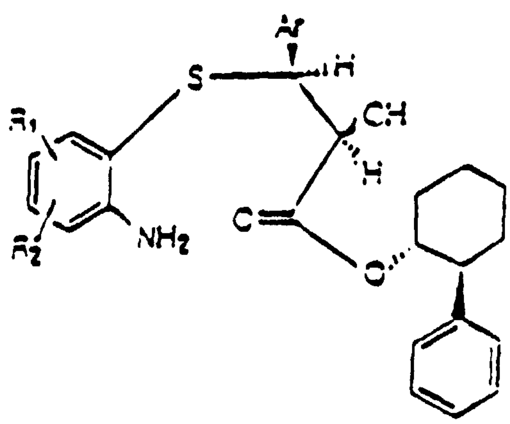

B

wherein $R_1$ and $R_2$ and Ar are as described above, by reacting same with a base in a polar, protic solvent; the compound of general formula A is cyclized to provide a compound of general formula C

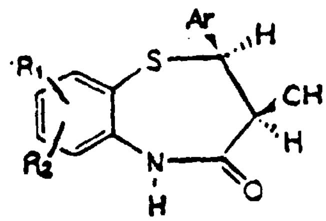

wherein $R_1$, $R_2$ and Ar are as described above. The cyclization is stated on page 4, lines 21 to 37 to be effected preferably in an organic solvent such as benzene, toluene, or more preferably a mixture of xylene at about the reflux termperature of the solvent for about 5 to 30 hours with nitrogen being bubbled through the reaction mixture and in the presence of an organic catalyst such as p-toluene sulphonic acid monohydrate. Also, EP-A-0 343 474 describes in Example 14B the cyclization of (2S,3S)-3-(2-aminophenyl)thio-2-hydroxy-3-(4-methoxybenzene)-propanoic acid with p-toluene sulphonic acid monohydrate with xylene as solvent with mixing at reflux under argon for 16 hours.

Also, EP-A-378 455 (which was published after the filing date of the present application) discloses a process for the preparation of a compound of the general formula

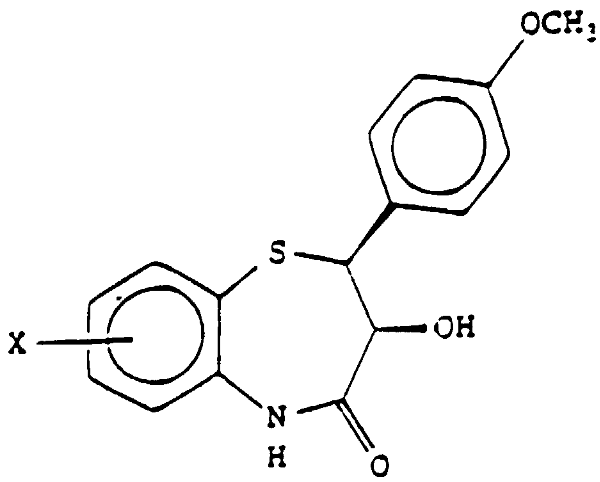

C

in which X represents one atom of hydrogen or chlorine, comprising firstly causing a 2-aminothiophenol of the general formula

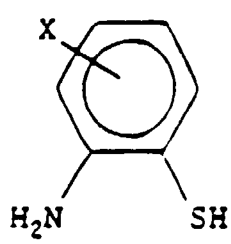

D

in which X is defined above, to react with (-)-(2R,3S)-2,3-epoxy-3-(4-methoxylproprionate of formula

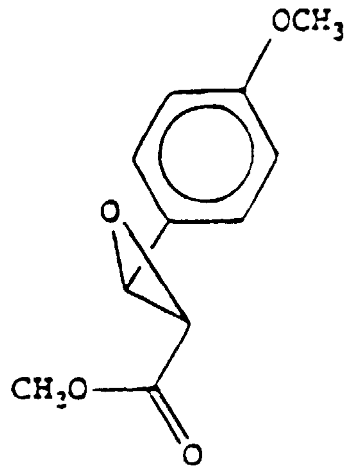

E

then cyclising the intermediate compound thus obtained, of general formula

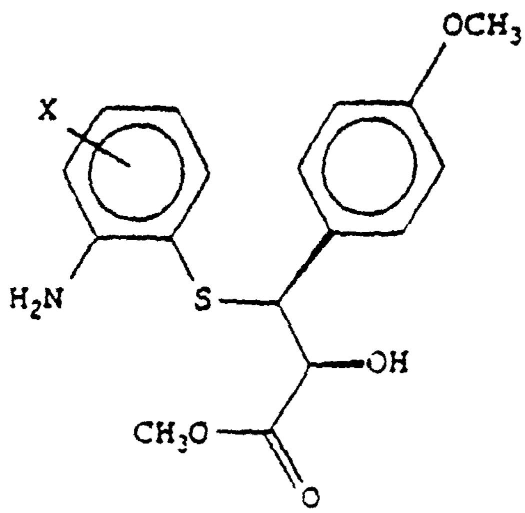

F

in the presence of acid, which process is characterised in that the two reaction steps are carried out, without isolation the intermediate compound, using a solvent chosen from among the chlorinated solvents having a boiling point above 70°C. The solvent used in these two reaction steps may be 1,2,3-trichloropropane, dichlorobenzene and is preferably chlorobenzene. The acid used to effect the cyclization may be anyone of a number of disclosed acids including p-toluene sulphonic acid, but is preferably methane sulphonic acid.

The present inventors have carried out various researches and as a result, they have found that, when the intramolecular ring closing reaction of a specific propionic acid compound (II) is carried out in the presence of a specific sulfonic acid compound, the reaction time can be remarkably shortened and the compound (I) can be prepared in good yield.

That is, according to the present invention there is provided a process for preparing 1,5-benzothiazepine derivatives represented by the formula:

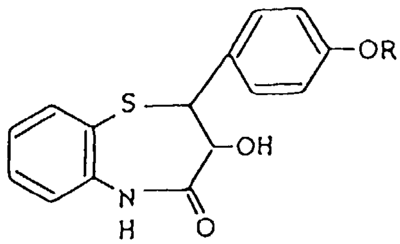

(I)

wherein R is a $C_{1-4}$ alkyl group,
which comprises subjecting a propionic acid compound represented by the formula:

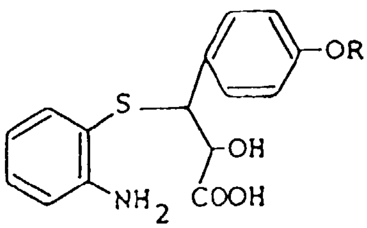

(II)

wherein R has the same meaning as defined above, to intramolecular ring closing reaction in the presence of a sulfonic acid compound represented by the formula:

$$R^1SO_3H \quad (III)$$

wherein $R^1$ represents a $C_{1-4}$ alkyl group or a phenyl group which may be substituted by at least one selected from methyl group, ethyl group, propyl group and butyl group;

provided that the intramolecular cyclization of the stereoisomer or a mixture of stereoisomers of 2-hydroxy-3-(2-aminophenyl)thio-3-(p-methoxyphenyl)-propionic acid is not carried out in an organic solvent such as benzene, toluene or xylene at reflux temperature with nitrogen being bubbled through the reaction mixture in the presence of an organic acid catalyst such as p-toluenesulfonic acid monohydrate, nor is it carried out in xylene at reflux temperature under argon in the presence of p-toluenesulfonic acid monohydrate.

Of the sulfonic acid compounds (III) to be used in the intramolecular ring closing reaction of the present invention preferred are methanesulfonic acid and p-toluenesulfonic acid. An amount of the said sulfonic acid is not particularly limited but generally, it is preferably used at an amount of 0.5 to 10 w/w %, more preferably about 1 to 6 w/w % based cn the compound (II).

The above reaction is preferably carried out in a high boiling point solvent such as xylene, toluene, etc. under reflux.

The desired compound (I) formed can be isolated as a pure product containing no sulfonic acid compound (III) by simple and easy operations as, for example, cooling the reaction mixture, collecting precipitated crystals by filtration, and washing them with a suitable solvent (e.g. ethanol, aqueous ethanol, etc.).

The invention also includes the use of a compound according to formula 1 produced in accordance with this invention in the production of the corresponding 3-acetoxy-5-(β-dimethylaminoethyl) -1,5-benzothiazepine derivatives represented by the formula:

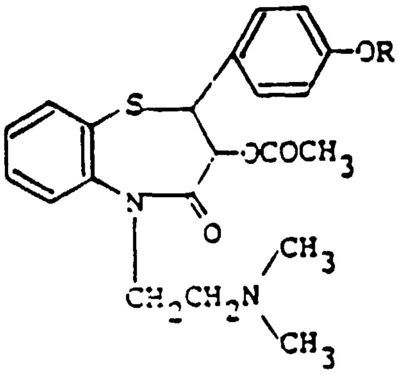

(IV)

wherein R is $C_1$-$C_4$ alkyl,
or a pharmaceutically acceptable salt thereof in a known method, for example, in a method described in Japanese Patent Publications (examined) No. 18038/1978 and No. 43785/1971 and U.S. Patents No. 3,562,257 and No. 4,438,035, the contents of which are hereby incorporated herein by reference.

According to the process of the present invention as mentioned above, a time required for the intramolecular ring closing reaction can be markedly shortened and the desired compound can be obtained in high yield and high purity. Therefore, the process of the present invention is excellent as an industrial process.

Throughout the specification and claims, the term "lower alkyl" should be interpreted as referring to an alkyl having one to four carbon atoms.

Experimental example

For forming (+)-cis-2-(4-methoxyphenyl)-3-hydroxy-2,3-dihydro-1,5-benzothiazepin-4(5H)-one by refluxing by heating 12.75 g of (+)-threo-2-hydroxy-3-(2-aminophenylthio)-3-(4-methoxyphenyl)propionic acid in 52 ml of xylene or toluene, under continuous removal of formed water, the effects of presence of a sulfonic acid compound in the reaction system were examined. The results are shown in Tables 1 and 2.

In the tables, the amount of the sulfonic acid compound is based on the starting compound, and p-toluenesulfonic acid is used as a monohydrate.

Table 1

(in xylene)

| | Sulfonic acid compound | Amount used (w/w %) | Reaction time | Yield of desired product (%) |
|---|---|---|---|---|
| Process of this invention | p-Toluenesulfonic acid | 5.4 | 30 min | 94 |
| | | 2.7 | 60 min | 93.9 |
| | | 1.1 | 1 hr & 15 min | 95.5 |
| | | 0.6 | 2 hrs & 15 min | 95.4 |
| | Methanesulfonic acid | 5.4 | 45 min | 93 |
| Control* | - | - | 12 hrs | 82.2 |

*: Control is Example described in Japanese Patent Publication No. 18038/1978

Table 2

(in toluene)

| | Sulfonic acid compound | Amount used (w/w %) | Reaction time | Yield of desired product (%) |
|---|---|---|---|---|
| Process of this invention | p-Toluenesulfonic acid | 5.4 | 5 hrs | 92 |
| | Methanesulfonic acid | 5.4 | 5 hrs | 92 |

Table 2 (continued)

| (in toluene) | | | | |
|---|---|---|---|---|
| | Sulfonic acid compound | Amount used (w/w %) | Reaction time | Yield of desired product (%) |
| Control | - | - | 7 hrs | < 50 |

As seen from the above Table 1 and Table 2, it can be understood that by the existance of p-toluenesulfonic acid or methanesulfonic acid in the reaction system, the desired product can be obtained within extremely short time in good yield.

Example 1

A mixture of 12.75 g of (+)-threo-2-hydroxy-3-(2-aminophenyl) thio -3-(4-methoxyphenyl)propionic acid, 140 mg of p-toluenesulfonic acid monohydrate and 52 ml of xylene is refluxed by heating for about 1 hour and 15 minutes. During the reaction, water formed is continuously removed. After cooling, precipitated crystals are collected by filtration and washed with cooled ethanol to give 11.5 g of (+)-cis-2-(4-methoxyphenyl)-3-hydroxy-2,3-dihydro-1,5-benzothiazepin-4(5H)-one.

Yield: 95.5 %

m.p.: 203 to 204 °C

$[\alpha]_D^{20}$ + 116 ° (c = 0.5, dimethylformamide)

Example 2

A mixture of 12.75 g of (+)-threo-2-hydroxy-3-(2-aminophenylthio)-3-(4-methoxyphenyl)propionic acid, 690 mg of p-toluenesulfonic acid monohydrate and 52 ml of toluene is refluxed by heating for about 5 hours. During the reaction, water formed is continuously removed. After cooling, precipitated crystals are collected by filtration and washed with cooled ethanol to give 11 g of (+)-cis-2-(4-methoxyphenyl)-3-hydroxy-2,3-dihydro-1,5-benzothiazepin-4(5H)-one.

Yield: 92 %

m.p.: 203 to 204.5 °C

$[\alpha]_D^{20}$ + 118 ° (c = 0.5, dimethylformamide)

## Claims

**1.** A process for preparing 1,5-benzothiazepine derivatives represented by the formula:

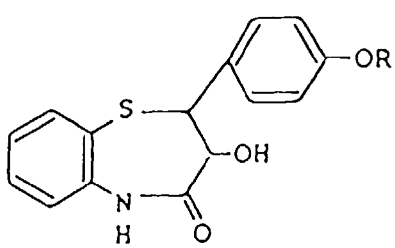

(I)

wherein R is a $C_{1-4}$ alkyl group,

which comprises subjecting a propionic acid compound represented by the formula:

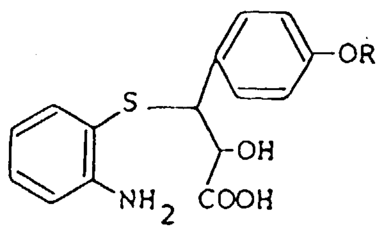

(II)

wherein R has the same meaning as defined above, to intramolecular ring closing reaction in the presence of a sulfonic acid compound represented by the formula:

$$R^1SO_3H \qquad (III)$$

wherein $R^1$ represents a $C_{1-4}$ alkyl group or a phenyl group which may be substituted by at least one selected from methyl group, ethyl group, propyl group and butyl group;

provided that the intramolecular cyclization of the stereoisomer or a mixture of stereoisomers of 2-hydroxy-3-(2-aminophenyl)thio-3-(p-methoxyphenyl)-propionic acid is not carried out in an organic solvent such as benzene, toluene or xylene at reflux temperature with nitrogen being bubbled through the reaction mixture in the presence of an organic acid catalyst such as p-toluenesulfonic acid monohydrate, nor is it carried out in xylene at reflux temperature under argon in the presence of p-toluenesulfonic acid monohydrate.

2. A process for preparing 1,5-benzothiazepine derivatives according to claim 1, wherein the sulfonic acid compound (III) is methanesulfonic acid or p-toluenesulfonic acid.

3. A process for preparing 1,5-benzothiazepine derivatives according to any one of the preceding claims, wherein the reaction is carried out in an appropriate solvent under reflux.

4. A process for preparing 1,5-benzothiazepine derivatives according to claim 3, wherein the solvent is at least one selected from xylene, toluene and dichlorobenzene.

5. A process for preparing 1,5-benzothiazepine derivatives according to any one of the preceding claims, wherein the sulfonic acid (III) is used in an amount of 0.5 to 10 w/w % based on the compound (II).

6. A process for preparing 1,5-benzothiazepine derivatives according to claim 5, wherein the sulfonic acid (III) is used in an amount of 1 to 6 w/w % based on the propionic acid compound (II).

7. A process for preparing 3-acetoxy-5-(β-dimethylaminoethyl)-1,5-benzothiazepine derivatives represented by the formula:

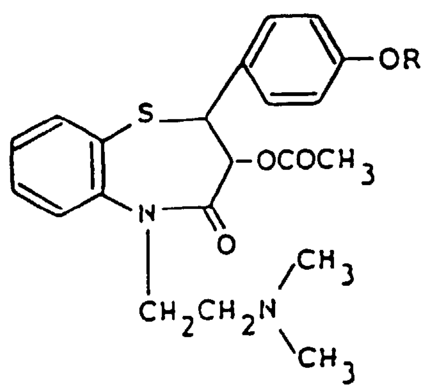

(IV)

wherein R is a $C_{1-4}$ alkyl group,
or pharmaceutically acceptable salt thereof which comprises the steps of:
subjecting a propionic acid compound represented by the formula:

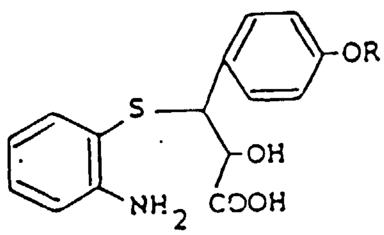

(II)

wherein R has the same meaning as defined above, to an intramolecular ring closing reaction in the presence of a sulfonic acid compound represented by the formula:

$$R^1SO_3H \qquad (III)$$

wherein $R^1$ represents a $C_{1-4}$ alkyl group or a phenyl group, which may be substituted by at least one selected from methyl group, ethyl group, propyl group and butyl group, to give a compound represented by the formula:

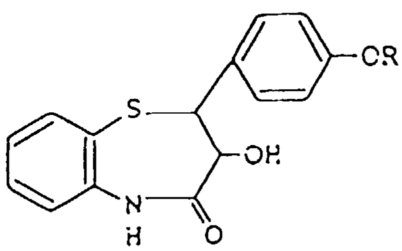

(I)

wherein R has the same meaning as defined above, and converting the compound (I) to the corresponding 3-acetoxy-5-(β-dimethylaminoethyl)-1,5-benzothiazepine derivative or a pharmaceutically acceptable salt thereof in a known method;
provided that the intramolecular cyclization of the stereoisomer or a mixture of stereoisomers of 2-hydroxy-3-(2-aminophenyl)thio-3-(p-methoxyphenyl)-propionic acid is not carried out in an organic solvent such as benzene, toluene or xylene at reflux temperature with nitrogen being bubbled through the reaction mixture in the presence of an organic acid catalyst such as p-toluenesulfonic acid monohydrate, nor is it carried out in xylene at reflux temperature under argon in the presence of p-toluenesulfonic acid monohydrate.

## Patentansprüche

**1.** Verfahren zur Herstellung von 1,5-Benzothiazepinderivaten, dargestellt durch die Formel:

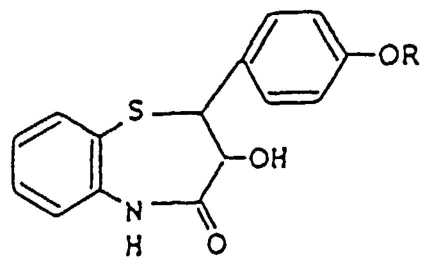

(I)

worin R eine $C_{1-4}$-Alkylgruppe darstellt,
welches umfaßt Unterziehen einer Propionsäureverbindung, dargestellt durch die Formel:

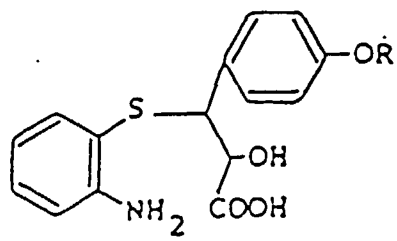

(II)

worin R die gleiche Bedeutung wie oben angegeben hat, einer intramolekularen Ringschlußreaktion in Gegenwart einer Sulfonsäureverbindung, dargestellt durch die Formel:

$$R^1SO_3H \qquad (III)$$

worin $R^1$ eine $C_{1-4}$-Alkylgruppe oder eine Phenylgruppe darstellt, welche mit mindestens einer Gruppe, ausgewählt aus einer Methylgruppe, Ethylgruppe, Propylgruppe und Butylgruppe substituiert sein kann;
unter der Voraussetzung, daß die intramolekulare Cyclisierung des Stereoisomers oder eines Gemisches von Stereoisomeren von 2-Hydroxy-3-(2-aminophenyl)thio-3-(p-methoxyphenyl)-propionsäure nicht in einem organischen Lösungsmittel wie etwa Benzol, Toluol oder Xylol bei Rückflußtemperatur ausgeführt wird, wobei Stickstoff durch das Reaktionsgemisch in Gegenwart eines organischen Säurekatalysators, wie etwa p-Toluolsulfonsäuremonohydrat geblubbert wird, noch daß sie in Xylol bei Rückflußtemperatur unter Argon in Gegenwart von p-Toluolsulfonsäuremonohydrat ausgeführt wird.

2. Verfahren zur Herstellung von 1,5-Benzothiazepinderivaten nach Anspruch 1, worin die Sulfonsäureverbindung (III) Methansulfonsäure oder p-Toluolsulfonsäure ist.

3. Verfahren zur Herstellung von 1,5-Benzothiazepinderivaten nach einem der vorhergehenden Ansprüche, worin die Reaktion in einem geeigneten Lösungsmittel unter Rückfluß ausgeführt wird.

4. Verfahren zur Herstellung von 1,5-Benzothiazepinderivaten nach Anspruch 3, worin das Lösungsmittel mindestens eines ausgewählt aus Xylol, Toluol und Dichlorbenzol ist.

5. Verfahren zur Herstellung von 1,5-Benzothiazepinderivaten nach einem der vorhergehenden Ansprüche, worin die Sulfonsäure (III) in einer Menge von 0,5 bis 10 Gew./Gew. %, bezogen auf die Verbindung (II) verwendet wird.

6. Verfahren zur Herstellung von 1,5-Benzothiazepinderivaten nach Anspruch 5, worin die Sulfonsäure (III) in einer Menge von 1 bis 6 Gew./Gew. %, bezogen auf die Propionsäureverbindung (II), verwendet wird.

7. Verfahren zur Herstellung von 3-Acetoxy-5-(β-dimethylaminoethyl)-1,5-benzothiazepinderivaten, dargestellt durch die Formel:

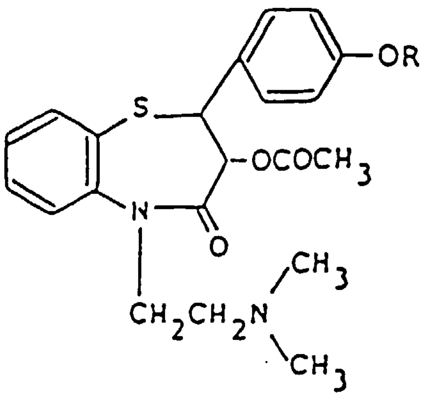

(IV)

worin R eine $C_{1-4}$-Alkylgruppe darstellt,
oder eines pharmazeutisch verträglichen Salzes davon, welches die Schritte umfaßt:
Unterziehen einer Propionsäureverbindung, dargestellt durch die Formel:

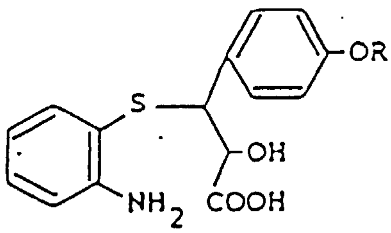

(II)

worin R die gleiche Bedeutung wie oben angegeben hat, einer intramolekularen Ringschlußreaktion in Gegenwart einer Sulfonsäureverbindung, dargestellt durch die Formel:

$$R^1SO_3H \quad \text{(III)}$$

worin $R^1$ eine $C_{1-4}$-Alkylgruppe oder eine Phenylgruppe darstellt, welche mit mindestens einer Gruppe, ausgewählt aus einer Methylgruppe, Ethylgruppe, Propylgruppe und Butylgruppe substituiert sein kann, um eine Verbindung zu ergeben, dargestellt durch die Formel:

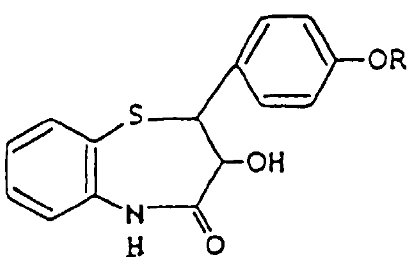

(I)

worin R die gleiche Bedeutung wie oben angegeben hat und Überführen der Verbindung (I) in das entsprechende 3-Acetoxy-5-(β-Dimethylaminoethyl)-1,5-benzothiazepinderivat oder ein pharmazeutisch verträgliches Salz davon auf eine bekannte Weise; unter der Voraussetzung, daß die intramolekulare Cyclisierung des Stereoisomers oder eines Gemisches von Stereoisomeren von 2-Hydroxy-3-(2-aminophenyl)thio-3-(p-methoxyphenyl)-propionsäure nicht in einem organischen Lösungsmittel wie etwa Benzol, Toluol oder Xylol bei Rückflußtemperatur ausgeführt wird, wobei Stickstoff durch das Reaktionsgemisch in Gegenwart eines organischen Säurekatalysators, wie etwa p-Toluolsulfonsäuremonohydrat geblubbert wird, noch daß sie in Xylol bei Rückflußtemperatur unter Argon in Gegenwart von p-Toluolsulfonsäuremonohydrat ausgeführt wird.

## Revendications

1. Procédé de préparation de dérivés de la 1,5-benothiazépine représentés par la formule suivante :

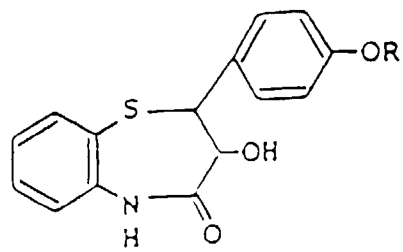

(I)

dans laquelle R représente un radical alkyle en $C_1$ à $C_4$,
caractérisé en ce que l'on soumet un composé de l'acide propionique représenté par la formule suivante :

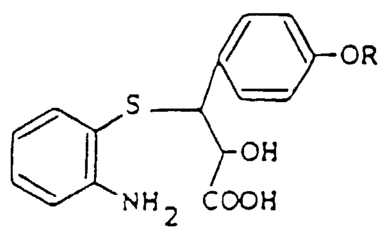

(II)

dans laquelle R possède les significations qui lui ont été attribuées ci-dessus,
à une réaction de fermeture de cycle intramoléculaire, en présence d'un composé d'un acide sulfonique représenté par la formule :

$$R^1SO_3H \qquad (III)$$

dans laquelle $R^1$ représente un radical alkyle en $C_1$ à $C_4$, ou un groupe phényle, qui peut être substitué par au moins un radical choisi parmi le groupe méthyle, le groupe éthyle, le groupe propyle et le groupe butyle,
avec la conditions que la cyclisation intramoléculaire du stéréoisomère, ou d'un mélange de stéréoisomères de l'acide 2-hydroxy-3-(2-aminophényl)thio-3-(p-méthoxyphényl)propionique ne soit pas entreprise dans un solvant organique comme le benzène, le toluène ou le xylène, à la température de reflux, de l'azote étant amené à barboter à travers le mélange réactionnel, en présence d'un catalyseur du type acide organique, comme l'acide p-toluènesulfonique monohydraté, ni non plus qu'elle soit entreprise dans du xylène, à la température de reflux, sous argon, en présence d'acide p-toluènesulfonique monohydraté.

2. Procédé de préparation de dérivés de la 1,5-benzothiazépine suivant la revendication 1, caractérisé en ce que le composé d'acide sulfonique (III) est l'acide méthanesulfonique, ou l'acide p-toluènesulfonique.

3. Procédé de préparation de dérivés de la 1,5-benzodiazépine suivant l'une quelconque des revendications précédentes, caractérisé en ce que l'on entreprend la réaction dans un solvant approprié au reflux.

4. Procédé de préparation de dérivés de la 1,5-benzothiazépine suivant la revendication 3, caractérisé en ce que le solvant est au moins l'un des composés suivants : xylène, toluène et dichlorobenzène.

5. Procédé de préparation de dérivés de la 1,5-benzodiazépine suivant l'une quelconque des revendications précédentes, caractérisé en ce que l'on utilise l'acide sulfonique (III), en une proportion de 0,5 à 10% p/p, sur base du composé (II).

**6.** Procédé de préparation de dérivés de la 1,5-benzothiazépine suivant la revendication 5, caractérisé en ce que l'on utilise l'acide sulfonique (III), en une proportion de 1 6% p/p, sur base du composé d'acide propionique (II).

**7.** Procédé de préparation de dérivés de la 3-acétoxy-5-(β-diméthylaminoéthyl)-1,5-benzothiazépine représentés par la formule :

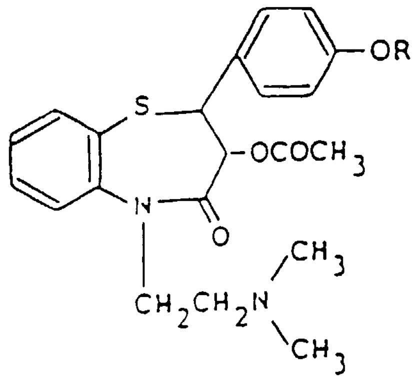

(IV)

dans laquelle R représente un radical alkyle en $C_1$ à $C_4$, ou d'un sel pharmaceutiquement acceptable de ces dérivés, caractérisé en ce qu'il comprend les étapes consistant à :
soumettre un composé d'acide propionique représenté par la formule :

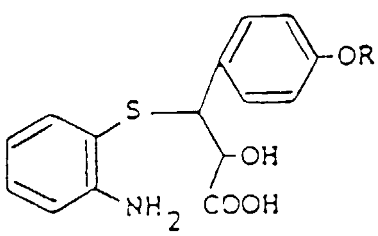

(II)

dans laquelle R possède les significations qui lui ont été attribuées ci-dessus,
à une réaction de fermeture de cycle intramoléculaire, en présence d'un composé d'acide sulfonique représenté par la formule suivante :

$$R^1SO_3H \quad (III)$$

dans laquelle $R^1$ représente un radical alkyle en $C_1$ à $C_4$, ou un groupe phényle, qui peut être substitué par au moins un radical choisi parmi le groupe méthyle, le groupe éthyle, le groupe propyle et le groupe butyle, de manière à obtenir un composé représenté par la formule suivante :

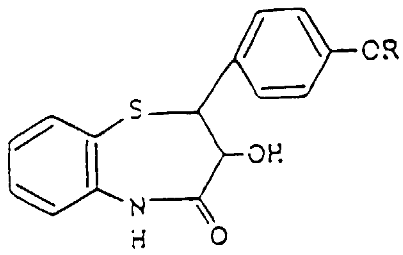

(I)

dans laquelle R possède les significations qui lui ont été attribuées ci-dessus,
et convertir le composé (I) en le dérivé de la 3-acétoxy-5-(β-diméthylaminoéthyl)-1,5-benzothiazépine ou en un sel pharmaceutiquement acceptable de celle-ci selon une méthode connue;
avec la condition que la cyclisation intramoléculaire du stéréoisomère ou d'un mélange de stéréoisomères de l'acide 2-hydroxy-3-(2-aminophényl)thio-3-(p-méthoxyphényl)propionique ne soit pas entreprise dans un solvant organique, comme le benzène, le toluène, ou le xylène, ou à la température de reflux, de l'azote étant amené à barboter à travers le mélange réactionnel, en présence d'un catalyseur du type acide organique, comme l'acide p-toluènesulfonique monohydraté, ni non plus qu'elle soit entreprise dans du xylène, à la température de reflux, sous argon, en présence d'acide p-toluènesulfonique monohydraté.